# EUROPEAN PATENT SPECIFICATION

(11) **EP 1 933 784 B1**
(45) Date of publication and mention of the grant of the patent: **21.12.2011**
(21) Application number: 06783895.3
(22) Date of filing: 18.08.2006
(51) Int. Cl.: A61F 6/04

(54) **CONDOM APPLICATOR**
KONDOM-APPLIKATOR
APPLICATEUR DE PRESERVATIF

(30) Priority: 19.08.2005 NL 1029771
(43) Date of publication of application: 25.06.2008
(60) Divisional application: 11180034.8
(73) Proprietor: MungosWorks Wingman B.V., 2628 AL Delft (NL)
(72) Inventor: TUNOVIC, Adnan, NL-2623 CD Delft (NL); BREUR, Paul, NL-2611 PT Delft (NL); KROEZEN, Marcel, NL-2623 CD Delft (NL); FOEKEMA, Raimond, Thomas, NL-2611 CN Delft (NL)
(74) Representative: Houben, Christiaan Hein Willem Frans
(86) International application number: PCT/NL2006/000428
(87) International publication number: WO 2007/021184

(56) References cited:
- EP-A- 0 429 144
- EP-A- 0 744 167
- WO-A-88/02624
- WO-A-97/00652
- US-A- 5 316 019
- US-B1- 6 425 397

## Description

The present invention relates to a condom applicator according to the preamble of claim 1.

Such a condom applicator is known from WO88/02624. Other examples of condom applicators are disclosed in EP429144 and US 5316019.

Yet another condom applicator is known from FR 2 693 652, which in Figures 3 and 4 discloses a condom applicator comprising a filamentary loop. The loop comprises two arcuate opposing guiding portions which in use are located at the rolled up part of the condom. Between the two guiding portions is provided a retention portion which in use lies on the rolled up part of the condom. At the ends of the loop, handling grips are provided.

In EP 744 167 a packaged condom is disclosed.

It is an object of the invention to provide an improved condom applicator.

This object is achieved by a condom applicator according claim 1. The condom applicator comprises at least one arcuate guiding member for engaging on the outside surface of a condom. The guiding member in use extends adjacent a rolled up part of said condom along at least a part of the circumference of the condom. On at least one end of said guiding member a receiving loop is provided for receiving the rolled up part of the condom. The receiving loop is open on the underside and extends in use over the rolled up part of the condom.

It is noted that in the above and in the following the underside is defined as the side where the open end of the condom is.

According to another aspect of the invention a condom applicator comprises two hingedly connected halves, each halve comprising a retention means for the rolled up part of the condom and a griping member for gripping each of the halves for moving them apart. The applicator halves can be hinged with respect to each other around a hinge axis which is parallel with the axis of the condom. The gripping members are preferably diametrically opposed, such that they can be readily gripped by fingers of one hand.

The invention will become more apparent from the following description with reference to the drawing, in which:
Fig. 1 shows a perspective view of a preferred embodiment of a condom applicator according to the invention with a rolled up condom arranged in it,
Fig. 2 shows in a schematic cross section the guiding of a condom in the condom applicator of Fig. 1,
Fig. 3 shows a perspective view of a slightly modified version of the embodiment of the condom applicator of Fig. 1,
Fig. 4 shows a packaged condom applicator of Fig. 1 with a condom prefitted in it,
Fig. 5 shows a perspective view of another embodiment of a condom applicator according to the invention,
Fig. 6a and 6b show a perspective view of yet another embodiment of a condom applicator according to the invention, and
Fig. 7a-7c show how a condom applicator according to the invention is handled in use.

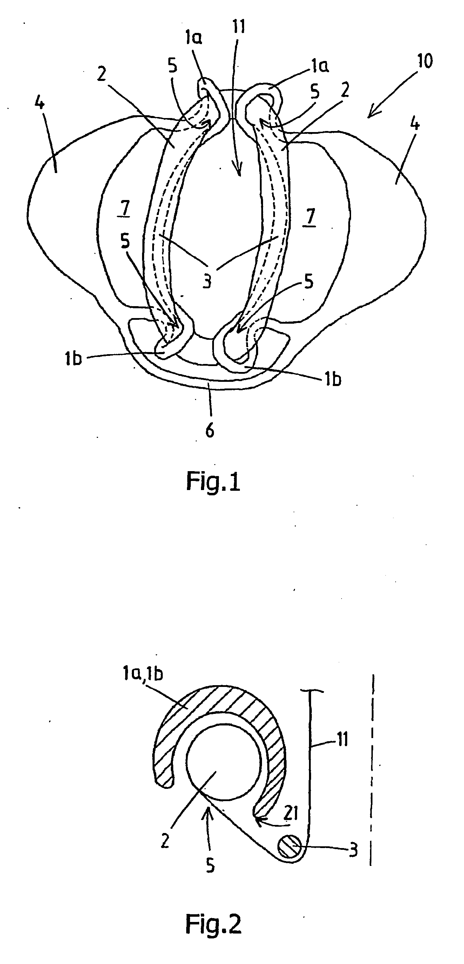

In Fig. 1 a condom applicator is shown which generally is indicated by reference numeral 10. A rolled up condom, indicated by reference numeral 11 is arranged in the condom applicator 10.

The condom applicator 10 has two opposing arcuate guiding members 3. The guiding members 3 in this embodiment are filamentary members with a round, preferably circular cross section. Each of the guiding members may describe an arc of a circle and is arranged such that it is directed with its concave side towards the opposing guiding member 3. Said arc of a circle preferably has a radius of curvature which is substantially greater than the average radius of a condom and the arc extends for example over an angle of 30° - 60°, preferably around 45°. This shape provides the advantage that the condom applicator 10 can be used to stretch condoms of different sizes, i.e. with different diameters.

Each end of the guiding members 3 is provided at its respective ends with a receiving loop 1a and 1b, respectively. The receiving loops 1a and 1b have a dimension such that a rolled up part 2 of the condom 11 in a fully rolled up state can be received in the receiving loops 1a, 1b of the respective guiding members 3. The receiving loops 1a, 1b have each an opening 5 which is narrower than the diameter, i.e. the thickness, of the rolled up part 2 of a fully rolled up condom 11.

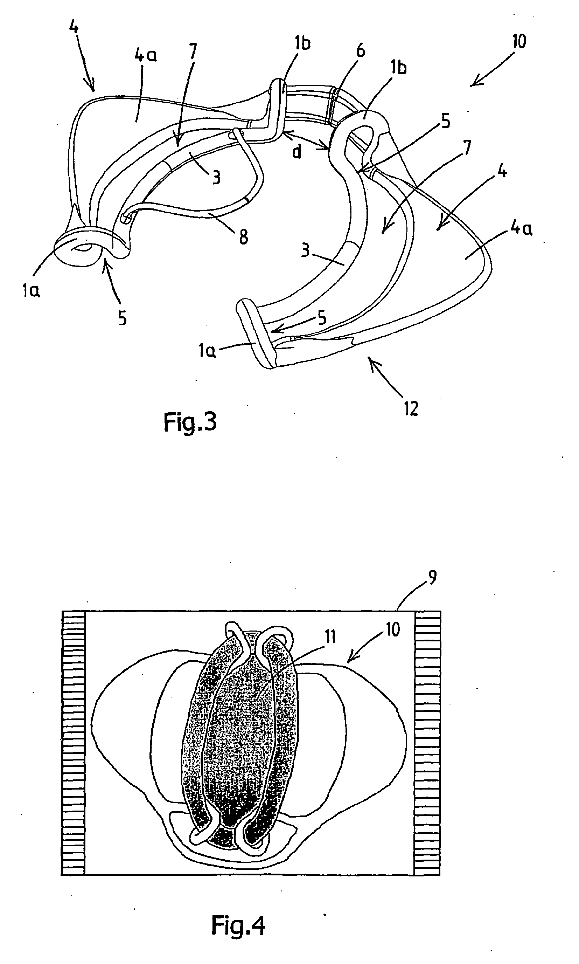

Each of the loops 1a, 1b extends from the corresponding guiding member 3 outwardly in a substantially radial direction with respect to the guiding member 3. In other words, each loop 1a, 1b lies in a plane which is substantially perpendicular with respect to the longitudinal direction of the corresponding guiding member 3 at the location where the loop 1a, 1b is arranged. This is best seen in Fig. 3. In this manner the loops 1a, 1b can enclose the rolled up part 2 of the condom 11- well and it is assured that the rolled up part of the condom 11 can rotate within the receiving loop 1.

In the preferred embodiment of Fig. 1 and Fig. 3 one end of each receiving loop 1a, 1b is attached to the corresponding guiding member 3. Preferably the receiving loops 1a, 1b are formed as an integral part of the guiding members 3. The other end of the receiving loop 1a, 1b is attached to a gripping member 4. The gripping member 4 is generally shaped as a ring segment which is situated radially outward from the guiding member. Each gripping member 4 extends substantially concentric with the corresponding guiding member 3. The ring segment can have a gripping tab 4a, which can have any suitable shape. Preferably the gripping tabs 4a are inclined in an upward and outward direction, which provides a better grip for moving the applicator 10 down along the penis. It is also possible to connect the gripping member 4 at another location to the guiding member 3. Between the gripping member 4 and the corresponding guiding member 3 a space 7 is present through which part of the condom 11 extends along the guiding member 3.

The guiding member 3, the receiving loops 1a, 1b attached to its ends and the gripping member 4 attached to the guiding member 3 via the receiving loops 1a, 1b, constitute one half of the condom applicator 10. The two opposing applicator halves are connected on one side by a hinge element 6, which defines a hinge axis which is parallel to the axis of the condom. The hinge element 6 comprises in the shown embodiment a flexible connection member, which is attached to one end of each of the guiding members 3 at the receiving loops 1a, 1b. The other ends of the guiding members 3 are free, i.e. not connected to each other and can be moved apart. The hinge member 6 is formed from a flexible material. It can be such that it acts as a spring, the spring force of which counteracts the spreading apart of the applicator halves, such that in an unloaded state the free end of the applicator halves are close together as is shown in Fig. 1 and Fig. 3.

The receiving loops 1a, 1b allow the condom 11 to be better retained by the applicator 10, also when the spreaded state of the applicator halves is varied during the application of the condom on a penis. In particular the loops 1a at the free end of the guiding elements 3, i.e. the end opposite to the end where the hinge element 6 is attached, are effective to retain the condom 11 and allow to determine better the moment at which the condom 11 is released by the applicator 10. This contrary to for example the condom applicator disclosed in FR 2 693 652, wherein there are no retention means at the open end of the loop and the condom can inadvertently be released too early from the applicator.

Preferably the loops 1b, which are at the end of the guiding elements 3 where the hinge element 6 is provided, are in an unloaded state of the applicator 10 spaced apart sufficiently to prevent that the condom 11 can get clamped between them, in particular when the applicator 10 is hinged open. The condom material getting stuck beteen the loops 1b would lead to a less smooth rolling off of the condom 11 and local stretching of the condom material resulting in stretching marks on the rolled of condom. It was found that with regard to the average size of condoms it was preferable to have a distance d (cf. Fig. 3) between the loops 1b of 0,5 - 0,8 mm to prevent this effect from occuring.

The condom applicator shown in Fig. 3 has a retention loop 8 which is attached with both its ends to one of the guiding members 3. The retention loop 8 is made of a flexible material and extends in the direction of the other guiding member 3 such that in use the teat part of a condom can extend through the retention loop 8 and is folded over it such that the end of the teat part can be laid under the opposing guiding member 3.

The condom applicator 10 in Figs. 1 and 3 are moulded in one piece from a flexible plastic material. A suitable material has sufficient elasticity such that the applicator 10 is elastically deformable, which is advantageous for its use. Furthermore, a suitable material is such that it has a sufficiently low roughness, such that the applicator 10 after the moulding process has a sufficiently smooth surface, which is important to prevent damage of the condom by the applicator 10. Polypropylene (PP) and High Density Polyethylene (HDPE) are found to fulfill the requirements, but also other suitable materials are conceivable.

The condom 11 can be arranged in the applicator 10 by bringing the rolled up condom 11 with its open end turned away from the applicator 10 from the underside (indicated in Fig. 3 by arrow 12) of the applicator 10 towards the applicator 10. The rolled up part 2 of the condom 11 is inserted through the openings 5 into the receiving loops 1a, 1b. By pressing the rolled up part 2 in the openings 5, which are narrower than the diameter of the rolled up part 2, the loops 1a, 1b, due to their flexibility deform and the openings become wider so as to allow the rolled up part 2 to pass. After the rolled up part 2 has passed the openings 5, the loops 1a, 1b deform back to their rest state, whereby the rolled up part 2 is retained in the four loops 1a, 1b of the condom applicator 10. The arcuate guiding members 3 are brought under the rolled up part 2 of the condom 11 as can be best seen in Fig. 1.

Although the design allows a user to readily fit a new condom in the applicator 10, it is at this moment envisaged that the condom applicator is a disposable product, which is thrown away after use. The condom applicator 10 and the condom 11 are preferably delivered in an preassembled state in one package 9 as is shown in Fig. 4, such that the assembly is immediately ready for use after removing the package 9. In this way no preparatory acts are necessary that could disturb the lovemaking.

When the condom is applied over a penis the diameter of the open end of the condom 11 can be increased by spreading the guiding members 3, by means of fingers of one hand gripping the opposed gripping members 4 and moving them apart. While moving down the condom applicator 10 along the penis the guiding members 3 guide the condom 11 over most of the circumference as is illustrated schematically in Fig. 2. Also, the guiding members 3 prevent that the condom 11 rubs against the edge 21 of the respective openings 5 of the loops 1a, 1b, which is illustrated in Fig. 2, which could lead to undesirable stretching of the condom material.

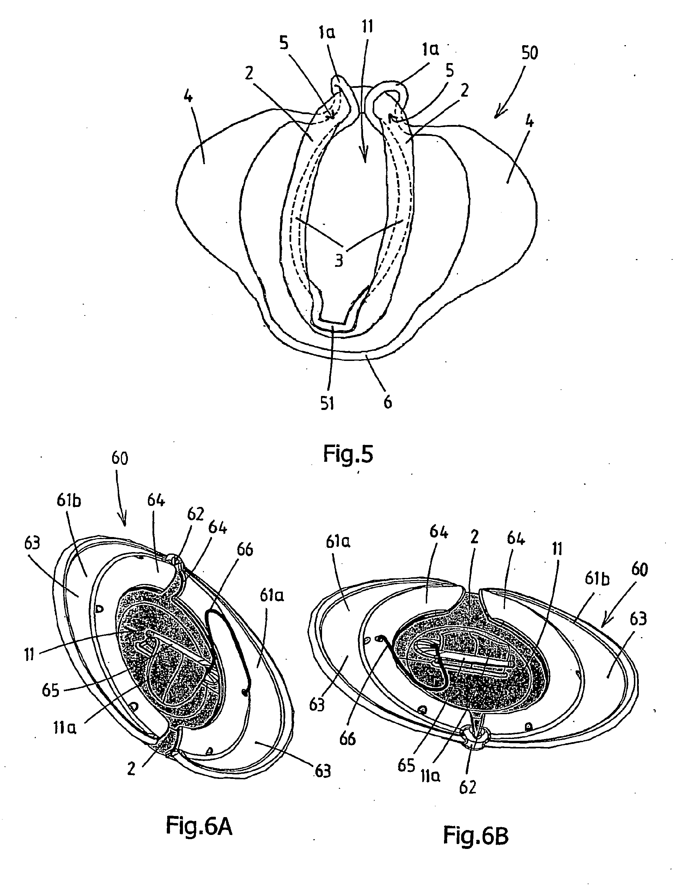

While moving down the applicator 10 along the penis, the rolled up part 2 of the condom 11 rolls within the receiving loops 1a, 1b. The working of the loops 1a, 1b is comparable with the working of a bearing. Because of the small contact surface of the loops 1a, 1b with the rolled up part 2 of the condom 11, a low friction between the condom 11 and the loops 1a, 1b is advantageously achieved during rolling off. This contrary to for example condom applicators which have a groove like receiving means (e.g. in Fig. 6) which contacts the rolled up part of the condom over a larger part of the circumference. The possible presence of a lubricant on the condom 11 can advantageously further reduce the bearing friction between the loops 1a, 1b and the rolled up part 2 of the condom 11. It is important that the rolled up part 2 of the condom 11 can roll in the loops 1a, 1b with a sufficiently low friction so as to prevent that the length rolled off of the rolled up part 2 does not become smaller than the length over which the applicator 10 is moved down along the penis, because this would lead to stretching of the condom material which creates undesired tensions.

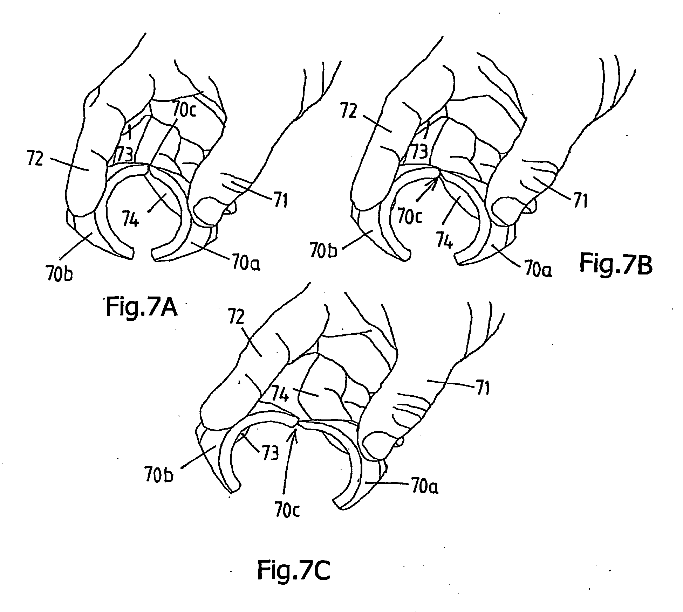

As a consequence of rolling down the rolled up part 2 of the condom 11, the diameter of rolled up part 2 decreases gradually. After the condom 11 has been unrolled over a certain length, the diameter of the rolled up part 2 will be smaller than the width of the respective openings 5 of the respective receiving loops 1a, 1b, such that the rolled up part 2 can pass through the said openings 5. Thus the applicator 10 can be removed from the condom 11. The handling of the applicator will be described further below with reference to Fig. 7a-7c.

In Fig. 5 a condom applicator 50 is shown which is a variant of the condom applicator of Fig. 1. Therefore corresponding elements are indicated by the same reference numerals. The condom applicator 50 only has loops 1a on one end of the respective guiding members 3. The other ends of the respective guiding members 3 are hingedly connected by a connection member 51. The connection member 51 can be formed as a filamentary member or another flexible member to allow a hinging between the applicator halves. The connection member engages the rolled up part 2 of the condom 11 on its upper side.

Still another embodiment of a condom applicator according to the invention is shown in Figs. 6a and 6b. This condom applicator indicated by reference numeral 60 comprises two applicator halves 61a and 61b, which are connected by a hinge member 62. Each applicator half 61a, 61b comprises a substantially semicircular ring segment. The radial innermost portion of the half ring has a receiving groove 64 which is open to the underside. In use the receiving groove 64 in the rolled up part 2 of a condom 11 is received. The radially outermost portion 63 of the ring segment constitutes a gripping surface which can be gripped between two fingers. A clip 65 which clamps on the teat portion 11a of the condom 11 is attached to the applicator half 61b by means of a cord 66 or the like. When applying the condom 11 on a penis the clip 65 will be pulled automatically from the teat portion 11a of the condom 11 by the action of the cord 66 which has only a limited length with respect to the full length of the condom 11.

The handling of the different embodiments of the condom applicator described in the above is now explained in general with reference to Figs. 7a-7c. In Figs. 7a-7c the condom is omitted for the sake of clarity. In Fig. 7a is shown how the gripping member of one applicator half 70a can be clamped between the thumb 71 and the ring finger 74. The gripping member of the other applicator half 70b can be clamped between the index finger 72 and the middle finger 73 of the same hand. Spreading the two mentioned pairs of fingers leads to the spreading of the applicator halves 70a and 70b, which are hingedly connected on one side by a hinge connection 70c.

By spreading the applicator halves 70a, 70b as is shown in Fig. 7b the rolled up part of the condom is stretched, such that it can be applied more easily over the penis and the applicator with the condom can be moved without much resistance in a quick movement downwards.

After the condom is fully rolled over the penis, the applicator halves 70a, 70b can be spread further as is shown in Fig. 7c, whereby on the side opposing the hinge connection an opening can be created which is broad enough for the penis to pass, such that the applicator can be removed in a lateral direction with respect to the penis.

It is noted that, although it is preferred, the gripping means do not necessarily have to be adapted to be gripped by two pairs of fingers. It is also envisaged that for instance an annular member or the like is provided at each applicator half. Each of said annular members is such that a finger can be inserted into it. By spreading the fingers, which are of the same hand, the applicator halves are spread and the same working is achieved as is described hereabove. Of course also other modifications of gripping members are imaginable, as long as the gripping members allow the applicator halves to be handled and be spread by the use of a single hand.

## Claims

1. Condom applicator (10, 50) comprising at least one arcuate guiding member (3) for engaging on the outside surface of a condom (11), which guiding member (3) in use extends adjacent a rolled up part (2) of said condom (11) along at least a part of the circumference of the condom (11), wherein on at least one end of said guiding member (3) a receiving loop (1a, 1b) is provided for receiving the rolled up part (2) of the condom (11), which receiving loop (1a, 1b) is open on the underside and extends in use over the rolled up part (2) of the condom (11), **characterized in that** the applicator (10, 50) comprises a plurality of mutually connected guiding members (3), which are connected by a hinge element (6), wherein a gripping member (4) is attached to each guiding member.

2. Condom applicator according to claim 1, wherein the guiding members (3) are moveable with respect to each other.

3. Condom applicator according to claim 1 or 2, wherein the applicator (10, 50) comprises two opposing guiding members (3), which are mutually connected, and which engage the condom (11) on opposite sides.

4. Condom applicator according to any one of the claims 1-3, wherein at both ends of the guiding members (3) a receiving loop (1a, 1b) is provided.

5. Condom applicator according to claim 3, wherein one end of the guiding members (3) is connected to the corresponding end of the opposing guide member (3) by a hinge element (6).

6. Condom applicator according to claim 5, wherein the hinge element (6) extends between the respective receiving loops (1b) at the end concerned of the respective guiding elements (3).

7. Condom applicator according to any one of the claims 1-6, wherein the hinge element (6) is constituted by a loop of flexible material.

8. Condom applicator according to any one of the claims 1-7, wherein the hinge element (6) has a spring action.

9. Condom applicator according to any one of the preceding claims, wherein the receiving loops (1a, 1b) extend transverse, preferably substantially perpendicular, to the longitudinal direction of the guiding element (3) at the end concerned.

10. Condom applicator according to claim 9, wherein the receiving loop (1a, 1b) is formed integrally with the corresponding guiding member (3).

11. Condom applicator according to claim 9 or 10, wherein the opening (5) in the receiving loops (1a, 1b) is directed substantially in the same direction as the open end of the condom (11).

12. Condom applicator according to any one of the preceding claims, wherein each gripping member (4) is located radially outward from the corresponding guiding member (3).

13. Condom applicator according to any one of the preceding claims, wherein the gripping member (4) is generally shaped as a ring segment which lies concentric with the corresponding arcuate guiding member (3).

14. Condom applicator according to claim 13, wherein the ring segment shaped gripping member (4) has a gripping tab (4a) extending outwardly substantially in a radial direction.

15. Condom applicator according to any one of the preceding claims, wherein the gripping member (4) is attached to both ends of the corresponding guiding member (3).

16. Condom applicator according to any one of the preceding claims, wherein the guiding members (3) are filamentary.

17. Condom applicator according to any one of the preceding claims, wherein the opening (5) in the receiving loop (1a, 1b) is narrower than the diameter of the rolled up part (2) of the condom (11) in its fully rolled up state.

18. Condom applicator according to any one of the preceding claims, wherein a retention means (8, 65) for the teat part (11a) of the condom (11) is provided.

19. Condom applicator according to claim 18, wherein the retention means (8, 65) for the teat part (11a) of the condom (11) is attached to at least one of the guiding members (3).

20. Condom applicator according to claim 19, wherein the retention means (8) is a flexible retention loop (8) which is attached with both its ends to one of the guiding members (3), which retention loop (8) extends in the direction of the other guiding member (3) such that in use the teat part (11a) of the condom (11) is extending through the retention loop (8) and is folded over it.

21. Condom applicator according to any one of the preceding claims, wherein the applicator (10, 50) is moulded in one piece from plastic.

22. Condom applicator according to any one of the preceding claims, wherein one of the gripping members (4, 63) is adapted to be clamped between the index finger and the middle finger and the other gripping member (4, 63) is adapted to be clamped between the thumb and the ring finger of the same hand.

23. Combination of a condom applicator (10, 50, 60) according to any one of the preceding claims and a condom (11).

24. Assembly of a condom applicator (10, 50, 60) according to any one of the claims 1-22 and a condom prefitted in the condom applicator (10, 50, 60).

25. Package (9) containing a condom applicator (10, 50, 60) according to any one of the claims 1-22 with a condom (11).

26. Package (9) according to claim 25, wherein the condom (11) is prefitted in the applicator (10, 50, 60).

## Patentansprüche

1. Kondom-Applikator (10, 50), umfassend zumindest ein bogenförmiges Führungselement (3) zum Eingreifen in die äußere Oberfläche eines Kondoms (11), wobei das Führungselement (3) sich bei Verwendung angrenzend an einen aufgerollten Abschnitt (2) des Kondoms (11) entlang zumindest eines Abschnittes des Umfangs des Kondoms (11) erstreckt, wobei an zumindest einem Ende des Führungselementes (3) eine Aufnehmschlaufe (1a, 1b) vorgesehen ist, um den aufgerollten Abschnitt (2) des Kondoms (11) aufzunehmen, wobei die Aufnehmschlaufe (1a, 1b) an der Unterseite offen ist und sich bei Verwendung über den aufgerollten Abschnitt (2) des Kondoms (11) erstreckt,
**dadurch gekennzeichnet, dass**
der Applikator (10, 50) eine Vielzahl an miteinander verbundenen Führungselementen (3) umfasst, die mittels eines Gelenkelementes (6) verbunden werden, wobei ein Greifelement (4) an jedem Führungselement befestigt ist.

2. Kondom-Applikator nach Anspruch 1, bei dem die Führungselemente (3) in Bezug aufeinander beweglich sind.

3. Kondom-Applikator nach Anspruch 1 oder 2, bei dem der Applikator (10, 50) zwei gegenüberliegende Führungselemente (3) umfasst, welche miteinander verbunden sind und welche mit dem Kondom (11) an gegenüberliegenden Seiten in Eingriff gelangen.

4. Kondom-Applikator nach einem der Ansprüche 1 bis 3, bei dem an beiden Enden der Führungselemente (3) eine Aufnehmschlaufe (1a, 1b) vorgesehen ist.

5. Kondom-Applikator nach Anspruch 3, bei dem ein Ende der Führungselemente (3) mit einem entsprechenden Ende des gegenüberliegenden Führungselementes (3) mittels eines Gelenkelementes (6) verbunden ist.

6. Kondom-Applikator nach Anspruch 5, bei dem das Gelenkelement (6) sich zwischen den jeweiligen Aufnehmschlaufen (1b) an dem Ende erstreckt, das mit den jeweiligen Führungselementen (3) betroffen ist.

7. Kondom-Applikator nach einem der Ansprüche 1 bis 6, bei dem das Gelenkelement (6) eine Schlaufe aus einem flexiblen Material umfasst.

8. Kondom-Applikator nach einem der Ansprüche 1 bis 7, bei dem das Gelenkelement eine Federwirkung aufweist.

9. Kondom-Applikator nach einem der vorhergehenden Ansprüche, bei dem die Aufnehmschlaufen (1a, 1b) sich quer, bevorzugt im Wesentlichen senkrecht, zu der Längsrichtung des Führungselementes (3) an dem betroffenen Ende erstrecken.

10. Kondom-Applikator nach Anspruch 9, bei dem die Aufnehmschlaufe (1a, 1b) einstückig mit dem entsprechenden Führungselement (3) ausgebildet ist.

11. Kondom-Applikator nach Anspruch 9 oder 10, bei dem die Öffnung (5) in den Aufnehmschlaufen (1a, 1b) in im Wesentlichen der gleichen Richtung wie das offene Ende des Kondoms (11) gerichtet ist.

12. Kondom-Applikator nach einem der vorhergehenden Ansprüche, bei dem jedes Greifelement (4) radial auswärts zu dem entsprechenden Führungselement (3) angeordnet ist.

13. Kondom-Applikator nach einem der vorhergehenden Ansprüche, bei dem das Greifelement (4) im Wesentlichen als ein Ringsegment geformt ist, welches konzentrisch zu dem entsprechenden bogenförmigen Führungselement (3) liegt.

14. Kondom-Applikator nach Anspruch 13, bei dem das ringsegmentförmige Greifelement (4) eine Greiflasche (4a) aufweist, die sich nach außen in im Wesentlichen einer radialen Richtung erstreckt.

15. Kondom-Applikator nach einem der vorhergehenden Ansprüche, bei dem das Greifelement (4) an beiden Enden des entsprechenden Führungselementes (3) befestigt ist.

16. Kondom-Applikator nach einem der vorhergehenden Ansprüche, bei dem die Führungselemente (3) faserartig sind.

17. Kondom-Applikator nach einem der vorhergehenden Ansprüche, bei dem die Öffnung der Aufnehmschlaufe (1a, 1b) schmäler ist als der Durchmesser des aufgerollten Abschnittes (3) des Kondoms (11) in seinem vollständig aufgerollten Zustand.

18. Kondom-Applikator nach einem der vorhergehenden Ansprüche, bei dem ein Rückhalteelement (8, 65) für den Reservoirabschnitt (11a) des Kondoms (11) vorgesehen ist.

19. Kondom-Applikator nach Anspruch 18, bei dem das Rückhalteelement (8, 65) für den Reservoirabschnitt (11a) des Kondoms (11) an zumindest einem der Führungselemente (3) befestigt ist.

20. Kondom-Applikator nach Anspruch 19, bei dem das Rückhalteelement (8) eine flexible Rückhaltschlaufe ist, die mit ihren beiden Enden an einem der Führungselemente (3) befestigt ist, wobei die Rückhalteschlaufe (80) in Richtung des anderen Führungselementes (3) sich so erstreckt, dass bei Verwendung der Reservoirabschnitt (11a) des Kondoms sich durch die Rückhalteschlaufe (8) erstreckt und über diese gefaltet ist.

21. Kondom-Applikator nach einem der vorhergehenden Ansprüche, bei dem der Applikator (10, 50) in einem Stück aus Plastik gegossen wird.

22. Kondom-Applikator nach einem der vorhergehenden Ansprüche, bei dem eines der Greifelemente (4, 63) ausgebildet ist, um zwischen den Zeigefinger und den Mittelfinger geklemmt zu werden und das andere Greifelement (4, 63) ausgebildet ist, um zwischen den Daumen und den Ringfinger derselben Hand geklemmt zu werden.

23. Kombination eines Kondom-Applikators (10, 50, 60) nach einem der vorhergehenden Ansprüche und eines Kondoms (11).

24. Anordnung eines Kondom-Applikators (10, 50, 60) nach einem der Ansprüche 1 bis 22 und eines Kondoms, welches vorab in den Kondom-Applikator (10, 50, 60) eingepasst ist.

25. Gebinde (9), umfassend einen Kondom-Applikator (10, 50, 60) nach einem der Ansprüche 1 bis 22, mit einem Kondom (11) .

26. Gebinde (9) nach Anspruch 25, bei dem das Kondom (11) vorab in den Applikator (10, 50, 60) eingepasst ist.

## Revendications

1. Applicateur de préservatif (10, 50) comprenant au moins un élément de guidage arqué (3) destiné à se mettre en prise sur la surface externe d'un préservatif (11), lequel élément de guidage (3), à l'usage, s'étend de manière adjacente à une partie enroulée (2) dudit préservatif (11) le long d'au moins une partie de la circonférence du préservatif (11), dans lequel sur au moins une extrémité dudit élément de guidage (3), on prévoit une boucle de réception (1a, 1b) pour recevoir la partie enroulée (2) du préservatif (11), laquelle boucle de réception (1a, 1 b) est ouverte sur la face inférieure et s'étend, à l'usage, sur la partie enroulée (2) du préservatif (11), **caractérisé en ce que** :
l'applicateur (10, 50) comprend une pluralité d'éléments de guidage (3) mutuellement raccordés, qui sont raccordés par un élément d'articulation (6), dans lequel un élément de préhension (4) est fixé à chaque élément de guidage.

2. Applicateur de préservatif selon la revendication 1, dans lequel les éléments de guidage (3) sont mobiles les uns par rapport aux autres.

3. Applicateur de préservatif selon la revendication 1 ou 2, dans lequel l'applicateur (10, 50) comprend deux éléments de guidage opposés (3) qui sont mutuellement raccordés, et qui mettent en prise le préservatif (11) sur les côtés opposés.

4. Applicateur de préservatif selon l'une quelconque des revendications 1 à 3, dans lequel au niveau des deux extrémités des éléments de guidage (3), on prévoit une boucle de réception (1a, 1 b).

5. Applicateur de préservatif selon la revendication 3, dans lequel une extrémité des éléments de guidage (3) est raccordée à l'extrémité correspondante de l'élément de guidage (3) opposé, par un élément d'articulation (6).

6. Applicateur de préservatif selon la revendication 5, dans lequel l'élément d'articulation (6) s'étend entre les boucles de réception (1 b) respectives au niveau de l'extrémité concernée des éléments de guidage (3) respectifs.

7. Applicateur de préservatif selon l'une quelconque des revendications 1 à 6, dans lequel l'élément d'articulation (6) est constitué par une boucle de matériau souple.

8. Applicateur de préservatif selon l'une quelconque des revendications 1 à 7, dans lequel l'élément d'articulation (6) a une action de rappel.

9. Applicateur de préservatif selon l'une quelconque des revendications précédentes, dans lequel les boucles de réception (1a, 1 b) s'étendent transversalement, de préférence sensiblement perpendiculairement à la direction longitudinale de l'élément de guidage (3) au niveau de l'extrémité concernée.

10. Applicateur de préservatif selon la revendication 9, dans lequel la boucle de réception (1a, 1b) est formée de manière solidaire avec l'élément de guidage (3) correspondant.

11. Applicateur de préservatif selon la revendication 9 ou 10, dans lequel l'ouverture (5) dans les boucles de réception (1a, 1b) est dirigée sensiblement dans la même direction que l'extrémité ouverte du préservatif (11).

12. Applicateur de préservatif selon l'une quelconque des revendications précédentes, dans lequel chaque élément de préhension (4) est positionné radialement vers l'extérieur de l'élément de guidage (3) correspondant.

13. Applicateur de préservatif selon l'une quelconque des revendications précédentes, dans lequel l'élément de préhension (4) est généralement formé comme un segment annulaire qui est concentrique avec l'élément de guidage (3) arqué correspondant.

14. Applicateur de préservatif selon la revendication 13, dans lequel l'élément de préhension (4) en forme de segment annulaire a une languette de préhension (4a) s'étendant vers l'extérieur sensiblement dans une direction radiale.

15. Applicateur de préservatif selon l'une quelconque des revendications précédentes, dans lequel l'élément de préhension (4) est fixé aux deux extrémités de l'élément de guidage (3) correspondant.

16. Applicateur de préservatif selon l'une quelconque des revendications précédentes, dans lequel les éléments de guidage (3) sont filamentaires.

17. Applicateur de préservatif selon l'une quelconque des revendications précédentes, dans lequel l'ouverture (5) dans la boucle de réception (1a, 1 b) est plus étroite que le diamètre de la partie enroulée (2) du préservatif (11) dans son état complètement enroulé.

18. Applicateur de préservatif selon l'une quelconque des revendications précédentes, dans lequel on prévoit des moyens de retenue (8, 65) pour la partie de réservoir (11a) du préservatif (11).

19. Applicateur de préservatif selon la revendication 18, dans lequel les moyens de retenue (8, 65) pour la partie de réservoir (11a) du préservatif (11) sont fixés à au moins l'un des éléments de guidage (3).

20. Applicateur de préservatif selon la revendication 19, dans lequel les moyens de retenue (8) sont une boucle de retenue souple (8) qui est fixée avec ses deux extrémités à l'un des éléments de guidage (3), laquelle boucle de retenue (8) s'étend dans la direction de l'autre élément de guidage (3) de sorte qu'à l'usage, la partie de réservoir (11a) du préservatif (11) s'étend à travers la boucle de retenue (8) et est repliée sur cette dernière.

21. Applicateur de préservatif selon l'une quelconque des revendications précédentes, dans lequel l'applicateur (10, 50) est moulé d'un seul tenant à partir d'une matière plastique.

22. Applicateur de préservatif selon l'une quelconque des revendications précédentes, dans lequel l'un des éléments de préhension (4, 63) est adapté pour être serré entre l'index et le majeur et l'autre élément de préhension (4, 63) est adapté pour être serré entre le pouce et l'annulaire de la même main.

23. Combinaison composée d'un applicateur de préservatif (10, 50, 60) selon l'une quelconque des revendications précédentes, et d'un préservatif (11).

24. Ensemble d'un applicateur de préservatif (10, 50, 60) selon l'une quelconque des revendications 1 à 22 et un préservatif pré-installé dans l'applicateur de préservatif (10, 50, 60).

25. Emballage (9) contenant un applicateur de préservatif (10, 50, 60) selon l'une quelconque des revendications 1 à 22 avec un préservatif (11).

26. Emballage (9) selon la revendication 25, dans lequel le préservatif (11) est pré-installé dans l'applicateur (10, 50, 60).
